# EUROPEAN PATENT SPECIFICATION

(11) **EP 2 188 393 B1**
(45) Date of publication and mention of the grant of the patent: **18.09.2013**
(21) Application number: 08785425.3
(22) Date of filing: 07.08.2008
(51) Int. Cl.: C12Q 1/68

(54) **PREDICTIVE MARKER FOR EGFR INHIBITOR TREATMENT**
PROGNOSEMARKER ZUR BEHANDLUNG MIT EGFR-HEMMERN
MARQUEUR PRÉDICTIF POUR UN TRAITEMENT PAR UN INHIBITEUR D'EGFR

(30) Priority: 14.08.2007 EP 07114314; 20.05.2008 EP 08156516
(43) Date of publication of application: 26.05.2010
(73) Proprietor: F. Hoffmann-La Roche AG, 4070 Basel (CH)
(72) Inventor: DELMAR, Paul, 4057 Basel (CH); KLUGHAMMER, Barbara, 79618 Rheinfelden (DE); LUTZ, Verena, 81371 München (DE); MCLOUGHLIN, Patricia, 4057 Basel (CH)
(74) Representative: Halbig, Dirk
(86) International application number: PCT/EP2008/006519
(87) International publication number: WO 2009/021680

(56) References cited:
- WO-A-01/94629
- WO-A-2004/071572
- WO-A-2005/049829
- WO-A-2006/101925
- WO-A-2006/119980
- WO-A-2007/067500
- MING-SOUND TSAO ET AL: "Erlotinib in Lung Cancer Molecular and Clinical Predictors of Outcome" NEW ENGLAND JOURNAL OF MEDICINE, MASSACHUSETTS MEDICAL SOCIETY, BOSTON, MA, US, vol. 353, no. 2, 14 July 2005 (2005-07-14), pages 133-145, XP007905868 ISSN: 1533-4406

### SEQUENCE LISTING

<110> F. Hoffmann-La Roche AG
<120> Predictive marker for EGFR inhibitor treatment
<130> 24413WO
<150> EP07114314.3
   <151> 2007-08-14
<150> EP08156516.0
   <151> 2008-05-20
<160> 4
<170> PatentIn version 3.4
<210> 1
   <211> 6629
   <212> DNA
   <213> Homo sapiens
<400> 1
   gctgcggccg cggctgaggg acctgcccgc gctgctgcgg agcgggctca cgctgcggag      60
   gaagcggagc gccgctgggg gccggactct atcaagaaga atatctaatc cgtaccttga     120
   acatacgcct tcccagattt atggagagaa ttcttcttgt gcaggaagag cattgaggaa     180
   tattattatc gttcaagcag ctgacctgat aaaggacaga gtgaacctca aggggtttta     240
   caggaggagc tgcgttgggt cagagctggt agactggctt ctagaacact gtcctttcgt     300
   ccagtgcaga tctatggcca taggagtctg gcaactccta ctggacatgg gaattatgtt     360
   atcagtggac cagcatctat actttcaaga tacttatgtt ttctaccagt tttcctctga     420
   tgaatgtagc tacttgtact gtgaatttga aagagaagaa gaatggcaaa atggtgtcaa     480
   gcttttactg caacttgtgc ctctcattcc tgccagaggt ggcatctgtg aactgtctca     540
   tcagaaaatt gaagactccg aagaaagcag tgatgaaatt cttgtgcgtc taacatctgc     600
   ggtgcagaga gagctagcag ctgttattgc tttgaaagca aggaagtctg caattgaaca     660
   agatgaagaa aacaacgaca aacatgtagc tgtaacagaa gccgaaagtg ttccagattc     720
   tcaggcaggg gtgatgtgca agctccagga aagagatgaa atcggacgaa ttgaactagt     780
   ccagaagctg gcaaaagaaa actatcagtt tttgcagacg gacaaaaaag aacaggagaa     840
   gtctgaacac caagatgatg aagtgacgac tgttcaggtt aaagagcaag accagagcgt     900
   cctggtgctg aagaaagtgc agtgctgtgg cccagccccc acagctggga gtgcggagag     960
   ccattggaga tatgtggtgg tgtccgggac cccggagaag attttggagc accttttgaa    1020
   tgacttgcac ctggaagaag tccaggacaa agaaacagag accctcctgg atgacttcct    1080
   tctcacgtac actgtcttca tgacaactga tgacttgtgc caggctctgt taaggcacta    1140
   ttctgctaag aagtatcaag gcaaagagga aaactcagac gttccgcgta ggaaacgtaa    1200
   agtcttgcat cttgtttccc agtggattgc tctgtacaaa gactggttac ctgaagatga    1260
   acattcaaaa atgtttttaa agaccatata taggaatgta ctggatgatg tttatgaata    1320
   tccaatactt gaaaaagaat tgaaagaatt tcaaaagata cttggaatgc accgtcgtca 1380
   cactgtagat gaatattcac cacaaaaaaa gaataaagcc cttttccacc aattcagtct 1440
   taaggagaac tggctccagc atagaggaac tgtgactgaa acggaggaaa ttttctgcca 1500
   cgtgtatata acagagcact cctatgtcag tgtgaaggca aaagtttcca gtatagccca 1560
   agagatccta aaagtcgtgg cagaaaagat ccagtatgca gaagaggatc tggctctggt 1620
   ggccatcaca ttctctgggg aaaagcatga acttcagcca aatgacttag tcatctccaa 1680
   atccctcgag gcatctggtc gaatatatgt ctaccggaaa gacctggcgg acactttgaa 1740
   cccatttgca gaaaatgagg aatcacagca aaggtcgatg aggattttgg gaatgaacac 1800
   ttgggatctt gctctggaat taatgaattt tgattggagt ctattcaatt caattcacga 1860
   gcaagagctg atctacttca cgttcagcag acagggaagt ggggaacaca ctgcaaatct 1920
   cagccttctg ctccagagat gcaatgaggt ccagctttgg gtggccacgg agattctgct 1980
   ctgcagccag ctgggcaagc gagtgcagct ggtgaaaaaa ttcatcaaaa ttgcggctca 2040
   ctgcaaagcc cagagaaacc tgaattcttt ctttgccatt gtgatgggtc tcaacactgc 2100
   ttctgtcagt cgactgtcgc agacctggga gaaaatccct gggaagttta agaaactttt 2160
   ctctgaactt gaaagtttaa cagatccttc cctaaatcac aaagcctaca gagatgcatt 2220
   caaaaagatg aagccaccaa aaatcccttt catgccctta ttgcttaaag atgtaacatt 2280
   tattcatgaa ggaaataaaa cttttttgga taatcttgtc aattttgaaa agctgcatat 2340
   gatcgcagac actgtccgaa ccctgagaca ctgcaggact aaccagtttg gtgacctgtc 2400
   tccaaaagag catcaagagt taaagtccta tgttaatcac ctgtatgtca ttgacagcca 2460
   gcaggctctg tttgagctct cacacaggat cgagcctcgg gtgtgagccc cactgcctca 2520
   cctcccctgt atctgcagca ctttgagcta cgggaatgtc tatgccaagc acgttgcttt 2580
   cctgtgagaa aagaagttgc tgagttttat cagtataacc caagacattc acaggaaagc 2640
   cagccaaagc gtgttcagga agtgatgtca gccaccagag agggggagag gtttctccat 2700
   gctactctcg ggacaagaag gcagaaggag agtcagaagc attcttgaga tggagaaggc 2760
   tggtttctta tgatcacatt gttgatccag tccagttttc aatatgagat gtgccagcat 2820
   caagacaaga caacgtcttg acatgcaatg accaaatatt tcattaagag cgtgcatgaa 2880
   acaggaagga gtttttactt tgcctagttt tagattactg tccataagct gtcaaagaag 2940
   tcattctttt gaacacctga tgacagagac agcatctcta gatctccagg gaggagaggt 3000
   ttctgttgat acaacctgtg acatcaccaa aagccacttg tgtctaggga gttagtgagg 3060
   actgcagcta gcatccatgc tctgatgggc agatgaacaa tgtcaaggtg tgcatcactt 3120
   tgcaccacaa tcaactattg acacatgctt gcaggtgaaa ttagtttctg tacaactgat 3180
   ttgcagctat aggcaaggta gatgaagttg ctttgccagt aaggaaaaat agtaatcttt 3240
   aagaaattga ctcattgttt aatttctggg gattttcttt atacttctaa gcaggctctt 3300
   atcttttatt ggacataata tgattttgaa aaagcacagt gcctgacaca ttgcaaacac 3360
   tcaccaactg cttgctgagg tgacagagtc acaaaagtct gcattcttgt gcctgatgat 3420
   gcattttgcg tacctcatac aggctccttg cccacactat ggaatgacag cagccagtgc 3480
   agggaggtta agtgacattt aatgagtgaa gcacttagca ctctctaggt aataagatag 3540
   tggtaattac tagtgttttg gcaaatgaaa aatgccctga aatagccaaa tgtctgatta 3600
   atgttggcaa cttagaagtc ctataatcca actaccagcc aaagcaggga gcctttctat 3660
   aatttgcctt tttttttttt tttttcaaaa tctgagtctt ctaaaatctt attattccca 3720
   tttttaccaa ttgaggctcc tgtagcaaat aagacctctt gatattttca aggactggtt 3780
   agaggatttc tttcaacctt cacatgaaca aaacagccta tgggtcaaaa taatgaaatc 3840
   cacccctgcc tgctagatac ttgtcacctt gctaaaatgc aagggcctgg tccattcatt 3900
   ttccaaatgc aggagtcttg gtgcacttct cactcttcct gcctgttcat ctctttcatg 3960
   cccacacaga cctgtttcct ttttgtctca tcaacgcctc attcatcctc attactgagg 4020
   cgtgtccaat gctttttgac atctttatag cagtgctgtt tcctgggctc aggaaccaca 4080
   ctgagcttga gatactgctg gaaggaacca tgtggagaga aggtttggga gaactttgag 4140
   agagacttag tttggcccag catgtaaaac ttcagtcctg aacatttata gggttttata 4200
   gaagggcatc ctccagggct ggtccattca gagaaatgct gcatgctgcc gtcatggaat 4260
   gtggcccaca ggacaccaga gccgtgagaa ccggagagca gacttccctc acggctgggc 4320
   tgagcaaacc ctccaaagcc ctcctcacgc agttactaac aatagcatgg gcttacagca 4380
   caagcacgtg ttctcacctt tttcctatgc cctggactaa ggtttggcca gtgtaatcat 4440
   ataaggccat cctgacattg tttctgtgtt tcaaaatttg gatttttatt tacattagaa 4500
   ctacattgct cctagtagaa cattaccttt aggggactaa ttttccatgg agaactattt 4560
   cagcatattg catgctgctc agaccccaag tcagatatgc ccaccaagcc agatgaagct 4620
   acacaaatgt ggtatttaaa tgcattttgt acagtgactt cagagtatct cacatgacat 4680
   gggtgtaaac tggctgggga gaaaatgatg cttgttcacc tcttcctcca gccgtggtta 4740
   ggtggtccta ggggtagcag agggaaggga ggattttgtg cagtcaagat ttgcttttcc 4800
   atccttgtct tctgaatgtc taaaatctct gcatctttct gaagtttaac aactgtctcc 4860
   agaggtttgc caggcagcag ctctcagaag tttccaaagc tttgcagaat cttagatctg 4920
   gaattaaaga attcaagccc gaattgtgag aaccagatat tactcaacag aaagctcttt 4980
   ctaaggaatc tgagctgttc actggtggac agtggtgggg cttgagtgct ccttgttaat 5040
   aggatgggcc atgcaccctc tctggatatt caccaaggcc tcttcagaat agggtttgtt 5100
   ctggctagaa gcgtggtcta gaagatggct aagctctttg ccagctctca tttggagttt 5160
   tattattgca taaaatcttc gctcactctg caaatcttac gtaatctggc accttcggca 5220
   ccaggtggtg caggggcact tctaagtggg ctctttttgt tacagcacaa ctctcagaca 5280
   gtcctgtggg tctttggatt cgtcagcatt ccagcaaact agccctgctt agaagttagc 5340
   acaagacagc agaatgcagg accccgtagg caaaatcaca accttgctat taaaaaaaat 5400
   tttgttttac atacacattt gcaggtgttc cctagagtgt ggtgttttga atttgctctt 5460
   tgtcatctgt ataattgcca aatgattata gtgatacaca tgacctgcat tcactttttt 5520
   ctagtttcct taattatgtt tagaataaat tcatttccct agaccgagaa ccacaaacag 5580
   gtagtgtgga gcatacaccg aatttagaag catgtggata aggtcagtgc tcacactgcc 5640
   tagtccacag ggagaggatg ctgcatgaat atatacttgc ctctgagtgg aggagaaatc 5700
   gtggcatgaa agagagagta ccagtgatga cttcttatcc ctggagctgg gctttcactg 5760
   ctacccatat cccagccctg cgagtctgtt ctagccagca cagacaccgc agatccggaa 5820
   ctgaatgttc ctaaatggcg cagccaatcc aggcttttca gaaactgggc aaaaacatta 5880
   aaatggggac gatcgggtct tccgcagtgg tccaacacag gatttctttt aaatgtttca 5940
   aaaacatgtc cttaaaattt cagcctgctt cttagcgagt gggccagttt tgcttaaaac 6000
   tggtgggggg gcggggggga agtttttaaa aattgccaaa aagttagatg caaatgtatt 6060
   actgtataaa gcaaagctgt atatactaaa cattttttag cagagtaata tttatttgca 6120
   tagtctattt attgtattcg tatcacactg ttattaaata ctgggatgaa atcagtgacc 6180
   tgaagcaaga aatcttgcct tttaatgtat cattaattag ggctgctgtg atattgtcag 6240
   cttgcattaa caattagaag atagagaacc cgccatcagg gtgtctacct aacttctcag 6300
   ggactacact tggtagtttt ccaccattta aagaactggt aaatatgaaa catttgttga 6360
   gttaccagaa ttgccattaa cagtgttttc tttcccatat tccatgcttt ctgcctctgt 6420
   gtatatatat aatatatatg tatatgactg tgctgtgtat ttatcgaagc tagtaagcaa 6480
   taatttatat gtaaaaatgg ccaagcaata taaggttaaa acttatataa gtaaccctta 6540
   ccttatcttg tattttcaat ttttttttaa aactgctttt ccaaatatga gactatgtta 6600
   aagacactat tcatgcctct taaaaaaaa 6629
<210> 2
   <211> 2619
   <212> DNA
   <213> Homo sapiens
<400> 2
   atgaaagcaa agcagtataa gctgccagcc ctccctcaag gagcttatgg attccttggg    60
   gcaaatggac acacagaaaa acagtcaaca atgctgagca cccaggtgct gcacctgaat   120
   agtagcaaaa acagagctct aaagaggagc gtaccccagc aggaggaact tcatgcagga   180
   gccgactcca caaaactgga cttcgagtct ggactagtca agtgtcagct tactcacacc   240
   gcttcctcga tttatggaga gaattcttct tgtgcaggaa gagcattgag gaatattatt   300
   atcgttcaag cagctgacct gataaaggac agagtgaacc tcaaggggtt ttacaggagg   360
   agctgcgttg ggtcagagct ggtagactgg cttctagaac actgtccttt cgtccagtgc   420
   agatctatgg ccataggagt ctggcaactc ctactggaca tgggaattat gttatcagtg   480
   gaccagcatc tatactttca agatacttat gttttctacc agttttcctc tgatgaatgt   540
   agctacttgt actgtgaatt tgaaagagaa gaagaatggc aaaatggtgt caagctttta   600
   ctgcaacttg tgcctctcat tcctgccaga ggtggcatct gtgaactgtc tcatcagaaa 660
   attgaagact ccgaagaaag cagtgatgaa attcttgtgc gtctaacatc tgcggtgcag 720
   agagagctag cagctgttat tgctttgaaa gcaaggaagt ctgcaattga acaagatgaa 780
   gaaaacaacg acaaacatgt agctgtaaca gaagccgaaa gtgttccaga ttctcaggca 840
   ggggtgatgt gcaagctcca ggaaagagat gaaatcggac gaattgaact agtccagaag 900
   ctggcaaaag aaaactatca gtttttgcag acggacaaaa aagaacagga gaagtctgaa 960
   caccaagatg atgaagtgac gactgttcag gttaaagagc aagaccagag cgtcctggtg 1020
   ctgaagaaag tgcagtgctg tggcccagcc cccacagctg ggagtgcgga gagccattgg 1080
   agatatgtgg tggtgtccgg gaccccggag aagattttgg agcacctttt gaatgacttg 1140
   cacctggaag aagtccagga caaagaaaca gagaccctcc tggatgactt ccttctcacg 1200
   tacactgtct tcatgacaac tgatgacttg tgccaggctc tgttaaggca ctattctgct 1260
   aagaagtatc aaggcaaaga ggaaaactca gacgttccgc gtaggaaacg taaagtcttg 1320
   catcttgttt cccagtggat tgctctgtac aaagactggt tacctgaaga tgaacattca 1380
   aaaatgtttt taaagaccat atataggaat gtactggatg atgtttatga atatccaata 1440
   cttgaaaaag aattgaaaga atttcaaaag atacttggaa tgcaccgtcg tcacactgta 1500
   gatgaatatt caccacaaaa aaagaataaa gcccttttcc accaattcag tcttaaggag 1560
   aactggctcc agcatagagg aactgtgact gaaacggagg aaattttctg ccacgtgtat 1620
   ataacagagc actcctatgt cagtgtgaag gcaaaagttt ccagtatagc ccaagagatc 1680
   ctaaaagtcg tggcagaaaa gatccagtat gcagaagagg atctggctct ggtggccatc 1740
   acattctctg gggaaaagca tgaacttcag ccaaatgact tagtcatctc caaatccctc 1800
   gaggcatctg gtcgaatata tgtctaccgg aaagacctgg cggacacttt gaacccattt 1860
   gcagaaaatg aggaatcaca gcaaaggtcg atgaggattt tgggaatgaa cacttgggat 1920
   cttgctctgg aattaatgaa ttttgattgg agtctattca attcaattca cgagcaagag 1980
   ctgatctact tcacgttcag cagacaggga agtggggaac acactgcaaa tctcagcctt 2040
   ctgctccaga gatgcaatga ggtccagctt tgggtggcca cggagattct gctctgcagc 2100
   cagctgggca agcgagtgca gctggtgaaa aaattcatca aaattgcggc tcactgcaaa 2160
   gcccagagaa acctgaattc tttctttgcc attgtgatgg gtctcaacac tgcttctgtc 2220
   agtcgactgt cgcagacctg ggagaaaatc cctgggaagt ttaagaaact tttctctgaa 2280
   cttgaaagtt taacagatcc ttccctaaat cacaaagcct acagagatgc attcaaaaag 2340
   atgaagccac caaaaatccc tttcatgccc ttattgctta aagatgtaac atttattcat 2400
   gaaggaaata aaactttttt ggataatctt gtcaattttg aaaagctgca tatgatcgca 2460
   gacactgtcc gaaccctgag acactgcagg actaaccagt ttggtgacct gtctccaaaa 2520
   gagcatcaag agttaaagtc ctatgttaat cacctgtatg tcattgacag ccagcaggct 2580
   ctgtttgagc tctcacacag gatcgagcct cgggtgtga 2619
<210> 3
   <211> 2619
   <212> DNA
   <213> Homo sapiens
<400> 3
   atgaaagcaa agcagtataa gctgccagcc ctccctcaag gagcttatgg attccttggg 60
   gcaaatggac acacagaaaa acagtcaaca atgctgagca cccaggtgct gcacctgaat 120
   agtagcaaaa acagagctct aaagaggagc gtaccccagc aggaggaact tcatgcagga 180
   gccgactcca caaaactgga cttcgagtct ggactagtca agtgtcagct tactcacacc 240
   gcttcctcga tttatggaga gaattcttct tgtgcaggaa gagcattgag gaatattatt 300
   atcgttcaag cagctgacct gataaaggac agagtgaacc tcaaggggtt ttacaggagg 360
   agctgcgttg ggtcagagct ggtagactgg cttctagaac actgtccttt cgtccagtgc 420
   agatctatgg ccataggagt ctggcaactc ctactggaca tgggaattat gttatcagtg 480
   gaccagcatc tatactttca agatacttat gttttctacc agttttcctc tgatgaatgt 540
   agctacttgt actgtgaatt tgaaagagaa gaagaatggc aaaatggtgt caagctttta 600
   ctgcaacttg tgcctctcat tcctgccaga ggtggcatct gtgaactgtc tcatcagaaa 660
   attgaagact ccgaagaaag cagtgatgaa attcttgtgc gtctaacatc tgcggtgcag 720
   agagagctag cagctgttat tgctttgaaa gcaaggaagt ctgcaattga acaagatgaa 780
   gaaaacaacg acaaacatgt agctgtaaca gaagccgaaa gtgttccaga ttctcaggca 840
   ggggtgatgt gcaagctcca ggaaagagat gaaatcggac gaattgaact agtccagaag 900
   ctggcaaaag aaaactatca gtttttgcag acggacaaaa aagaacagga gaagtctgaa 960
   caccaagatg atgaagtgac gactgttcag gttaaagagc aagaccagag cgtcctggtg 1020
   ctgaagaaag tgcagtgctg tggcccagcc cccacagctg ggagtgcgga gagccattgg 1080
   agatatgtgg tggtgtccgg gaccccggag aagattttgg agcacctttt gaatgacttg 1140
   cacctggaag aagtccagga caaagaaaca gagaccctcc tggatgactt ccttctcacg 1200
   tacactgtct tcatgacaac tgatgacttg tgccaggctc tgttaaggca ctattctgct 1260
   aagaagtatc aaggcaaaga ggaaaactca gatgttccgc gtaggaaacg taaagtcttg 1320
   catcttgttt cccagtggat tgctctgtac aaagactggt tacctgaaga tgaacattca 1380
   aaaatgtttt taaagaccat atataggaat gtactggatg atgtttatga atatccaata 1440
   cttgaaaaag aattgaaaga atttcaaaag atacttggaa tgcaccgtcg tcacactgta 1500
   gatgaatatt caccacaaaa aaagaataaa gcccttttcc accaattcag tcttaaggag 1560
   aactggctcc agcatagagg aactgtgact gaaacggagg aaattttctg ccacgtgtat 1620
   ataacagagc actcctatgt cagtgtgaag gcaaaagttt ccagtatagc ccaagagatc 1680
   ctaaaagtcg tggcagaaaa gatccagtat gcagaagagg atctggctct ggtggccatc 1740
   acattctctg ggaaaaagca tgaacttcag ccaaatgact tagtcatctc caaatccctc 1800
   gaggcatctg gtcgaatata tgtctaccgg aaagacctgg cggacacttt gaacccattt 1860
   gcagaaaatg aggaatcaca gcaaaggtcg atgaggattt tgggaatgaa cacttgggat 1920
   cttgctctgg aattaatgaa ttttgattgg agtctattca attcaattca cgagcaagag 1980
   ctgatctact tcacgttcag cagacaggga agtggggaac acactgcaaa tctcagcctt 2040
   ctgctccaga gatgcaatga ggtccagctt tgggtggcca cggagattct gctctgcagc 2100
   cagctgggca agcgagtgca gctggtgaaa aaattcatca aaattgcggc tcactgcaaa 2160
   gcccagagaa acctgaattc tttctttgcc attgtgatgg gtctcaacac tgcttctgtc 2220
   agtcgactgt cgcagacctg ggagaaaatc cctgggaagt ttaagaaact tttctctgaa 2280
   cttgaaagtt taacagatcc ttccctaaat cacaaagcct acagagatgc attcaaaaag 2340
   atgaagccac caaaaatccc tttcatgccc ttattgctta aagatgtaac atttattcat 2400
   gaaggaaata aaactttttt ggataatctt gtcaattttg aaaagctgca tatgatcgca 2460
   gacactgtcc gaaccctgag acactgcagg actaaccagt ttggtgacct gtctccaaaa 2520
   gagcatcaag agttaaagtc ctatgttaat cacctgtatg tcattgacag ccagcaggct 2580
   ctgtttgagc tctcacacag gatcgagcct cgggtgtga 2619
<210> 4
   <211> 2619
   <212> DNA
   <213> Homo sapiens
<400> 4
   atgaaagcaa agcagtataa gctgccagcc ctccctcaag gagcttatgg attccttggg      60
   gcaaatggac acacagaaaa acagtcaaca atgctgagca cccaggtgct gcacctgaat     120
   agtagcaaaa acagagctct aaagaggagc gtaccccagc aggaggaact tcatgcagga     180
   gccgactcca caaaactgga cttcgagtct ggactagtca agtgtcagct tactcacacc     240
   gcttcctcga tttatggaga gaattcttct tgtgcaggaa gagcattgag gaatattatt     300
   atcgttcaag cagctgacct gataaaggac agagtgaacc tcaaggggtt ttacaggagg     360
   agctgcgttg ggtcagagct ggtagactgg cttctagaac actgtccttt cgtccagtgc     420
   agatctatgg ccataggagt ctggcaactc ctactggaca tgggaattat gttatcagtg     480
   gaccagcatc tatactttca agatacttat gttttctacc agttttcctc tgatgaatgt     540
   agctacttgt actgtgaatt tgaaagagaa gaagaatggc aaaatggtgt caagctttta     600
   ctgcaacttg tgcctctcat tcctgccaga ggtggcatct gtgaactgtc tcatcagaaa     660
   attgaagact ccgaagaaag cagtgatgaa attcttgtgc gtctaacatc tgcggtgcag     720
   agagagctag cagctgttat tgctttgaaa gcaaggaagt ctgcaattga acaagatgaa     780
   gaaaacaacg acaaacatgt agctgtaaca gaagccgaaa gtgttccaga ttctcaggca     840
   ggggtgatgt gcaagctcca ggaaagagat gaaatcggac gaattgaact agtccagaag     900
   ctggcaaaag aaaactatca gtttttgcag acggacaaaa aagaacagga gaagtctgaa     960
   caccaagatg atgaagtgac gactgttcag gttaaagagc aagaccagag cgtcctggtg    1020
   ctgaagaaag tgcagtgctg tggcccagcc cccacagctg ggagtgcgga gagccattgg   1080
   agatatgtgg tggtgtccgg gaccccggag aagattttgg agcacctttt gaatgacttg   1140
   cacctggaag aagtccagga caaagaaaca gagaccctcc tggatgactt ccttctcacg   1200
   tacactgtct tcatgacaac tgatgacttg tgccaggctc tgttaaggca ctattctgct   1260
   aagaagtatc aaggcaaaga ggaaaactca gatgttccgc gtaggaaacg taaagtcttg   1320
   catcttgttt cccagtggat tgctctgtac aaagactggt tacctgaaga tgaacattca   1380
   aaaatgtttt taaagaccat atataggaat gtactggatg atgtttatga atatccaata   1440
   cttgaaaaag aattgaaaga atttcaaaag atacttggaa tgcaccgtcg tcacactgta   1500
   gatgaatatt caccacaaaa aaagaataaa gcccttttcc accaattcag tcttaaggag   1560
   aactggctcc agcatagagg aactgtgact gaaacggagg aaattttctg ccacgtgtat   1620
   ataacagagc actcctatgt cagtgtgaag gcaaaagttt ccagtatagc ccaagagatc   1680
   ctaaaagtcg tggcagaaaa gatccagtat gcagaagagg atctggctct ggtggccatc   1740
   acattctctg gggaaaagca tgaacttcag ccaaatgact tagtcatctc caaatccctc   1800
   gaggcatctg gtcgaatata tgtctaccgg aaagacctgg cggacacttt gaacccattt   1860
   gcagaaaatg aggaatcaca gcaaaggtcg atgaggattt tgggaatgaa cacttgggat   1920
   cttgctctgg aattaatgaa ttttgattgg agtctattca attcaattca cgagcaagag   1980
   ctgatctact tcacgttcag cagacaggga agtggggaac acactgcaaa tctcagcctt   2040
   ctgctccaga gatgcaatga ggtccagctt tgggtggcca cggagattct gctctgcagc   2100
   cagctgggca agcgagtgca gctggtgaaa aaattcatca aaattgcggc tcactgcaaa   2160
   gcccagagaa acctgaattc tttctttgcc attgtgatgg gtctcaacac tgcttctgtc   2220
   agtcgactgt cgcagacctg ggagaaaatc cctgggaagt ttaagaaact tttctctgaa   2280
   cttgaaagtt taacagatcc ttccctaaat cacaaagcct acagagatgc attcaaaaag   2340
   atgaagccac caaaaatccc tttcatgccc ttattgctta aagatgtaac atttattcat   2400
   gaaggaaata aaactttttt ggataatctt gtcaattttg aaaagctgca tatgatcgca   2460
   gacactgtcc gaaccctgag acactgcagg actaaccagt ttggtgacct gtctccaaaa   2520
   gagcatcaag agttaaagtc ctatgttaat cacctgtatg tcattgacag ccagcaggct   2580
   ctgtttgagc tctcacacag gatcgagcct cgggtgtga                          2619

## Description

The present invention provides a biomarker that is predictive for the response to treatment with an EGFR inhibitor in cancer patients

A number of human malignancies are associated with aberrant or over-expression of the epidermal growth factor receptor (EGFR). EGF, transforming growth factor α (TGF-α), and a number of other ligands bind to the EGFR, stimulating autophosphorylation of the intracellular tyrosine kinase domain of the receptor. A variety of intracellular pathways are subsequently activated, and these downstream events result in tumour cell proliferation in vitro. It has been postulated that stimulation of tumour cells via the EGFR may be important for both tumour growth and tumour survival in vivo.

Early clinical data with Tarceva™, an inhibitor of the EGFR tyrosine kinase, indicate that the compound is safe and generally well tolerated at doses that provide the targeted effective concentration (as determined by preclinical data). Clinical phase I and II trials in patients with advanced disease have demonstrated that Tarceva™ has promising clinical activity in a range of epithelial tumours. Indeed, Tarceva™ has been shown to be capable of inducing durable partial remissions in previously treated patients with head and neck cancer, and NSCLC (Non small cell lung cancer) of a similar order to established second line chemotherapy, but with the added benefit of a better safety profile than chemo therapy and improved convenience (tablet instead of intravenous [i.v.] administration). A recently completed, randomised, double-blind, placebo-controlled trial (BR.21) has shown that single agent Tarceva™ significantly prolongs and improves the survival of NSCLC patients for whom standard therapy for advanced disease has failed.

Tarceva™ (erlotinib) is a small chemical molecule; it is an orally active, potent, selective inhibitor of the EGFR tyrosine kinase (EGFR-TKI).

Lung cancer is the major cause of cancer-related death in North America and Europe. In the United States, the number of deaths secondary to lung cancer exceeds the combined total deaths from the second (colon), third (breast), and fourth (prostate) leading causes of cancer deaths combined. About 75% to 80% of all lung cancers are non-small cell lung cancer (NSCLC), with approximately 40% of patients presenting with locally advanced and/or unresectable disease. This group typically includes those with bulky stage IIIA and IIIB disease, excluding malignant pleural effusions.

The crude incidence of lung cancer in the European Union is 52.5, the death rate 48.7 cases1100000/year. Among men the rates are 79.3 and 78.3, among women 21.6 and 20.5, respectively. NSCLC accounts for 80% of all lung cancer cases. About 90% of lung cancer mortality among men, and 80% among women, is attributable to smoking.

In the US, according to the American Cancer Society, during 2004, there were approximately 173,800 new cases of lung cancer (93,100 in men and 80,700 in women) and were accounting for about 13% of all new cancers. Most patients die as a consequence of their disease within two years of diagnosis. For many NSCLC patients, successful treatment remains elusive. Advanced tumours often are not amenable to surgery and may also be resistant to tolerable doses of radiotherapy and chemotherapy. In randomized trials the currently most active combination chemotherapies achieved response rates of approximately 30% to 40% and a 1-year survival rate between 35% and 40%. This is really an advance over the 10% 1-year survival rate seen with supportive care alone (Shepherd 1999).

Until recently therapeutic options for patients following relapse were limited to best supportive care or palliation. A recent trial comparing docetaxel (Taxotere^{®}) with best supportive care showed that patients with NSCLC could benefit from second line chemotherapy after cisplatin-based first-line regimens had failed. Patients of all ages and with ECOG performance status of 0, 1, or 2 demonstrated improved survival with docetaxel, as did those who had been refractory to prior platinum-based treatment. Patients who did not benefit from therapy included those with weight loss of > 10%, high lactate dehydrogenase levels, multi-organ involvement, or liver involvement. Additionally, the benefit of docetaxel monotherapy did not extend beyond the second line setting. Patients receiving docetaxel as third-line treatment or beyond showed no prolongation of survival. Single-agent docetaxel became a standard second-line therapy for NSCLC. Recently another randomized phase III trial in second line therapy of NSCLC compared pemetrexed (Alimta^{®}) with docetaxel. Treatment with pemetrexed resulted in a clinically equivalent efficacy but with significantly fewer side effects compared with docetaxel.

It has long been acknowledged that there is a need to develop methods of individualising cancer treatment. With the development of targeted cancer treatments, there is a particular interest in methodologies which could provide a molecular profile of the tumour target, (i.e. those that are predictive for clinical benefit). Proof of principle for gene expression profiling in cancer has already been established with the molecular classification of tumour types which are not apparent on the basis of current morphological and immunohistochemical tests.

WO2006/101925 (28.9.2006) discloses methods for determining which patients will most benefit from treatment with an epidermal growth factor receptor (EGFR) kinase inhibitor.

Therefore, it is an aim of the present invention to provide expression biomarkers that are predictive for the response to EGFR inhibitor treatment in cancer patients.

In a first object the present invention provides an in vitro method of predicting the response of a NSCLC patient to treatment with erlotinib comprising the steps: determining an mRNA expression level of a RAPGEF5 gene in a tumour sample of a patient and comparing the expression level of the RAPGEF5 gene to a value representative of an expression level of the RAPGEF5 gene in tumours of a non responding patient population, wherein a higher expression level of the at least one gene in the tumour sample of the patient is indicative for a patient who will respond to the treatment.

The abbreviation RAPGEF5 means Rap guanine nucleotide exchange factor (GEF) 5. The nucleotide sequence of the human RAPGEF5 gene and of its transcript variants 1, 6 and 11 are given in Seq. Id. Nos. 1 to 4. The gene bank accession numbers of the RAPGEF5 gene and of its variants and the corresponding Sequence identification numbers (Seq. Id. No.) are listed in table 4.

The term "a value representative of an expression level of the RAPGEF5 gene in tumours of a non responding patient population" refers to an estimate of the mean expression level of the marker gene in tumours of a population of patients who do not respond to the treatment with the EGFR inhibitor.

In a preferred embodiment, the marker gene RAPGEF5 shows typically between 1.5 and 2.7 or more fold higher expression in the tumour sample of the responding patient compared to the value representative of an expression level of the RAPGEF5 gene in tumours of a non responding patient population.

In a preferred embodiment, the expression level of the RAPGEF5 gene is determined by microarray technology or other technologies that assess RNA expression levels like quantitative RT-PCR or, although not part of the invention, by any method looking at the expression level of the respective protein, eg immunohistochemistry (IHC). The construction and use of gene chips are well known in the art. see, U. S. Pat Nos. 5,202,231; 5,445,934; 5,525,464; 5,695,940; 5,744,305; 5,795, 716 and 1 5,800,992. See also, Johnston, M. Curr. Biol. 8:R171-174 (1998); Iyer VR et al., Science 283:83-87 (1999). Of course, the gene expression level can be determined by other methods that are known to a person skilled in the art such as e.g. northern blots, RT-PCR, real time quantitative PCR, primer extension, RNase protection, RNA expression profiling.

The gene of the present invention can be combined to biomarker sets with other predictive biomarkers. Biomarker sets can be built from any combination of predictive biomarkers to make predictions about the effect of EGFR inhibitor treatment in cancer patients. The various biomarkers and biomarkers sets described herein can be used, for example, to predict how patients with cancer will respond to therapeutic intervention with an EGFR inhibitor.

The term "gene" as used herein comprises variants of the gene. The term "variant" relates to nucleic acid sequences which are substantially similar to the nucleic acid sequences given by the GenBank accession number. The term "substantially similar" is well understood by a person skilled in the art. In particular, a gene variant may be an allele which shows nucleotide exchanges compared to the nucleic acid sequence of the most prevalent allele in the human population. Preferably, such a substantially similar nucleic acid sequence has a sequence similarity to the most prevalent allele of at least 80%, preferably at least 85%, more preferably at least 90%, most preferably at least 95%. The term "variants" is also meant to relate to splice variants.

In some aspects, the EGFR inhibitor can be selected from the group consisting of gefitinib, erlotinib, PKI-166, EKB-569, GW2016, CI-1033 and an anti-erbB antibody such as trastuzumab and cetuximab.

In embodiments of the invention, the EGFR inhibitor is erlotinib and the cancer is NSCLC.

Techniques for the detection of gene expression of the genes described by this invention include, but are not limited to northern blots, RT-PCR, real time quantitative PCR, primer extension, RNase protection, RNA expression profiling and related techniques. These techniques are well known to those of skill in the art see e.g. Sambrook J et al., Molecular Cloning: A Laboratory Manual, Third Edition (Cold Spring Harbor Press, Cold Spring Harbor, 2000).

Techniques for the detection of protein expression of the respective genes described by this disclosure include, but are not limited to immunohistochemistry (IHC).

In accordance with the invention, cells from a patient tissue sample, e.g., a tumour or cancer biopsy, can be assayed to determine the expression pattern of one or more biomarkers. Success or failure of a cancer treatment can be determined based on the biomarker expression pattern of the cells from the test tissue (test cells), e.g., tumour or cancer biopsy, as being relatively similar or different from the expression pattern of a control set of the one or more biomarkers. In the context of this invention, it was found that the gene listed in table 3 is upregulated i.e. shows a higher expression level, in tumours of patients who respond to the EGFR inhibitor treatment compared to tumours of patients who do not respond to the EGFR inhibitor treatment. Thus, if the test cells show a biomarker expression profile which corresponds to that of a patient who responded to cancer treatment, it is highly likely or predicted that the individual's cancer or tumour will respond favourably to treatment with the EGFR inhibitor. By contrast, if the test cells show a biomarker expression pattern corresponding to that of a patient who did not respond to cancer treatment, it is highly likely or predicted that the individual's cancer or tumour will not respond to treatment with the EGFR inhibitor.

The marker gene of the present invention can be combined with other biomarkers to biomarker sets. Biomarker sets can be built from any combination of predictive biomarkers to make predictions about the effect of EGFR inhibitor treatment in cancer patients. The biomarkers and biomarkers sets described herein can be used, for example, to predict how patients with cancer will respond to therapeutic intervention with an EGFR inhibitor.

In a further object the present invention provides erlonitib for use in treating a NSCLC patient identified by the in vitro method of the present invention. Said therapeutic method comprises administering erlotinib to the patient who has been selected for treatment based on the predictive expression pattern of the RAPGEF5 gene listed in table 3. The EGFR inhibitor is erlotinib and the cancer to be treated is NSCLC.

### Short description of the figures

Figure 1 shows the study design;
Figure 2 shows the scheme of sample processing;
Figure 3a shows RAPGEF5 expression levels versus clinical outcome for GeneChip^{®} profiling;
Figure 3b shows RAPGEF5 expression levels versus clinical outcome for qRT-PCR and
Figure 3c shows the correlation between Genechip^{®} and qRT-PCR measurements for RAPGEF5.

### Experimental part

### Rationale for the Study and Study Design

Recently mutations within the EGFR gene in the tumour tissue of a subset of NSCLC patients and the association of these mutations with sensitivity to erlotinib and gefitinib were described (Pao W, et al. 2004; Lynch et al. 2004; Paez et al. 2004). For the patients combined from two studies, mutated EGFR was observed in 13 of 14 patients who responded to gefitinib and in none of the 11 gefitinib-treated patients who did not respond. The reported prevalence of these mutations was 8% (2 of 25) in unselected NSCLC patients. These mutations were found more frequently in adenocarcinomas (21 %), in tumours from females (20%), and in tumours from Japanese patients (26%). These mutations result in increased in vitro activity of EGFR and increased sensitivity to gefitinib. The relationship of the mutations to prolonged stable disease or survival duration has not been prospectively evaluated.

Based on exploratory analyses from the BR.21 study, it appeared unlikely that the observed survival benefit is only due to the EGFR mutations, since a significant survival benefit is maintained even when patients with objective response are excluded from analyses (data on file). Other molecular mechanisms must also contribute to the effect.

Based on the assumption that there are changes in gene expression levels that are predictive of response / benefit to Tarceva™ treatment, microarray analysis was used to detect these changes

This required a clearly defined study population treated with Tarceva™ monotherapy after failure of 1st line therapy. Based on the experience from the BR.21 study, benefiting population was defined as either having objective response, or disease stabilization for ≥ 12 weeks. Clinical and microarray datasets were analyzed according to a pre-defined statistical plan.

The application of this technique requires fresh frozen tissue (FFT). Therefore a mandatory biopsy had to be performed before start of treatment. The collected material was frozen in liquid nitrogen (N₂).

A second tumour sample was collected at the same time and stored in paraffin (formalin fixed paraffin embedded, FFPE). This sample was analysed for alterations in the EGFR signalling pathway.

The ability to perform tumour biopsies via bronchoscopy was a prerequisite for this study. Bronchoscopy is a standard procedure to confirm the diagnosis of lung cancer. Although generally safe, there is a remaining risk of complications, e.g. bleeding.

This study was a first step towards an individualized therapy for patients with refractory NSCLC. This individualized therapy will allow treating physicians to select the most appropriate agent out of the existing drugs for this indication.

Once individualized therapy will be available, the benefit for each future patient will outweigh the risk patients have to take in the present study:
response rates / number of benefiting patients will increase,
the risk of adverse side effects due to ineffective treatment will be reduced.

### Rationale for Dosage Selection

Tarceva™ was given orally once per day at a dose of 150 mg until disease progression, intolerable toxicities or death. The selection of this dose was based on pharmacokinetic parameters, as well as the safety and tolerability profile of this dose observed in Phase I, II and III trials in heavily pre-treated patients with advanced cancer. Drug levels seen in the plasma of patients with cancer receiving the 150 mg/day dose were consistently above the average plasma concentration of 500 ng / ml targeted for clinical efficacy. BR.21 showed a survival benefit with this dose.

### Objectives of the Study

The primary objective was the identification of differentially expressed genes that are predictive for benefit (CR, PR or SD ≥ 12 weeks) of Tarceva™ treatment. Identification of differentially expressed genes predictive for "response" (CR, PR) to Tarceva™ treatment was an important additional objective.

The secondary objectives were to assess alterations in the EGFR signalling pathways with respect to benefit from treatment.

### Study Design

### Overview of Study Design and Dosing Regimen

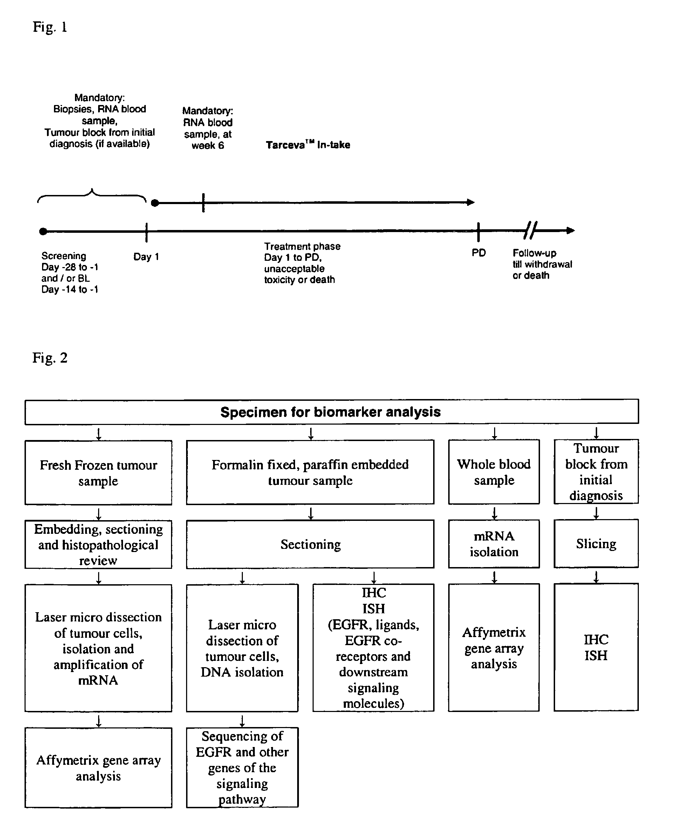

This was an open-label, predictive marker identification Phase II study. The study was conducted in approximately 26 sites in about 12 countries. 264 patients with advanced NSCLC following failure of at least one prior chemotherapy regimen were enrolled over a 12 month period. Continuous oral Tarceva™ was given at a dose of 150 mg/day. Dose reductions were permitted based on tolerability to drug therapy. Clinical and laboratory parameters were assessed to evaluate disease control and toxicity. Treatment continued until disease progression, unacceptable toxicity or death. The study design is depicted in figure 1.

Tumour tissue and blood samples were obtained for molecular analyses to evaluate the effects of Tarceva™ and to identify subgroups of patients benefiting from therapy.

### Predictive Marker Assessments

Biopsies of the tumour were taken within 2 weeks before start of treatment. Two different samples were collected:
The first sample was always frozen immediately in liquid N₂
The second sample was fixed in formalin and embedded in paraffin
Snap frozen tissue had the highest priority in this study.
Figure 2 shows a scheme of the sample processing.

### Microarray Analysis

The snap frozen samples were used for laser capture microdissection (LCM) of tumour cells to extract tumour RNA and RNA from tumour surrounding tissue. The RNA was analysed on Affymetrix microarray chips (HG-U133A) to establish the patients' tumour gene expression profile. Quality Control of Affymetrix chips was used to select those samples of adequate quality for statistical comparison.

### Single Biomarker Analyses on Formalin Fixed Paraffin Embedded Tissue

The second tumour biopsy, the FFPE sample, was used to perform DNA mutation, IHC and ISH analyses as described below. Similar analyses were performed on tissue collected at initial diagnosis.

The DNA mutation status of the genes encoding EGFR and other molecules involved in the EGFR signalling pathway were analysed by DNA sequencing. Gene amplification of EGFR and related genes were be studied by FISH.

Protein expression analyses included immunohistochemical [IHC] analyses of EGFR and other proteins within the EGFR signalling pathway.

### Response Assessments

The RECIST (Uni-dimensional Tumour Measurement) criteria were used to evaluate response. These criteria can be found under the following link:http://www.eortc.be/recist/

Note that:To be assigned a status of CR or PR, changes in tumour measurements must be confirmed by repeated assessments at least 4 weeks apart at any time during the treatment period.

In the case of SD, follow-up measurements must have met the SD criteria at least once after study entry at a minimum interval of 6 weeks.

In the case of maintained SD, follow-up measurements must have met the SD criteria at least once after study entry with maintenance duration of *at least* 12 weeks.

### Survival Assessment

A regular status check every 3 months was performed either by a patient's visit to the clinic or by telephone. All deaths were recorded. At the end of the study a definitive confirmation of survival was required for each patient.

### Response to erlotinib treatment

A total of 264 patients from 12 countries and 26 centres were enrolled in the study. 26% had Stage IIIB and 24% Stage IV NSCLC. 13.6% (n=36) of patients achieved an objective response while 31.4% (n=83) had clinical benefit (defined as having either an objective response or stable disease for 12 weeks or more). Median overall survival was 7.6 (CI 7-9) months and median progression-free survival was 11.3 (CI 8-12) weeks. Full details about the clinical data are shown in Table 1.

Fresh frozen bronchoscopic biopsies were collected from all subjects, but either not all samples had sufficient tumour content prior to microdissection (LCM) or did not have sufficient RNA yield after LCM to proceed to microarray analysis, so that tumour material was only available for 125 patients; 122 of these had evaluable RNA. Another set of 20 samples did not pass our quality control assessment of the microarray data. Of the 102 microarray data sets that were suitable for statistical analysis, the clinical characteristics are shown in Table 1. While 36 patients in the overall study achieved an objective response, only 6 of these had microarray data; similarly for those achieving clinical benefit the number of subjects with microarray data was only 21 as compared to 83 in the full data set. 6 were judged to be partial responders (PR), 31 had SD and 49 had PD; of the 6 patients with a PR, 5 had adenocarcinoma and one had squamous cell carcinoma. There were no patients achieving a CR in the data set.

### Methods

### RNA sample preparation and quality control of RNA samples

All biopsy sample processing was handled by a pathology reference laboratory; fresh frozen tissue samples were shipped from investigator sites to the Clinical Sample Operations facility in Roche Basel and from there to the pathology laboratory for further processing. Laser capture microdissection was used to select tumour cells from surrounding tissue. After LCM, RNA was purified from the enriched tumour material. The pathology laboratory then carried out a number of steps to make an estimate of the concentration and quality of the RNA..

RNases are RNA degrading enzymes and are found everywhere and so all procedures where RNA will be used must be strictly controlled to minimize RNA degradation. Most mRNA species themselves have rather short half-lives and so are considered quite unstable. Therefore it is important to perform RNA integrity checks and quantification before any assay.

RNA concentration and quality profile can be assessed using an instrument from Agilent (Agilent Technologies, Inc., Palo Alto, CA) called a 2100 Bioanalyzer®. The instrument software generates an RNA Integrity Number (RIN), a quantitation estimate (Schroeder, A., et al., *The RIN: an RNA integrity number for assigning integrity values to RNA measurements.* BMC Mol Biol, 2006. 7: p. 3), and calculates ribosomal ratios of the total RNA sample. The RIN is determined from the entire electrophoretic trace of the RNA sample, and so includes the presence or absence of degradation products.

The RNA quality was analysed by a 2100 Bioanalyzer®. Only samples with at least one rRNA peak above the added poly-I noise and sufficient RNA were selected for further analysis on the Affymetrix platform. The purified RNA was forwarded to the Roche Centre for Medical Genomics (RCMG; Basel, Switzerland) for analysis by microarray. 122 RNA samples were received from the pathology laboratory for further processing.

### Target Labeling of tissue RNA samples

Target labeling was carried out according to the Two-Cycle Target Labeling Amplification Protocol from Affymetrix (Affymetrix, Santa Clara, California), as per the manufacturer's instructions.

The method is based on the standard Eberwine linear amplification procedure but uses two cycles of this procedure to generate sufficient labeled cRNA for hybridization to a microarray.

Total RNA input used in the labeling reaction was 10ng for those samples where more than 10ng RNA was available; if less than this amount was available or if there was no quantity data available (due to very low RNA concentration), half of the total sample was used in the reaction. Yields from the labeling reactions ranged from 20-180µg cRNA. A normalization step was introduced at the level of hybridization where 15µg cRNA was used for every sample.

Human Reference RNA (Stratagene, Carlsbad, CA, USA) was used as a control sample in the workflow with each batch of samples. 10ng of this RNA was used as input alongside the test samples to verify that the labeling and hybridization reagents were working as expected.

### Microarray hybridizations

Affymetrix HG-U133A microarrays contain over 22,000 probe sets targeting approximately 18,400 transcripts and variants which represent about 14,500 well-characterized genes.

Hybridization for all samples was carried out according to Affymetrix instructions (Affymetrix Inc., Expression Analysis Technical Manual, 2004). Briefly, for each sample, 15µg of biotin-labeled cRNA were fragmented in the presence of divalent cations and heat and hybridized overnight to Affymetrix HG-U133A full genome oligonucleotide arrays. The following day arrays were stained with streptavidin-phycoerythrin (Molecular Probes; Eugene, OR) according to the manufacturer's instructions. Arrays were then scanned using a GeneChip Scanner 3000 (Affymetrix), and signal intensities were automatically calculated by GeneChip Operating Software (GCOS) Version 1.4 (Affymetrix).

### Statistical Analysis

Analysis of the Affymetrix™ data consisted of four main steps.

Step 1 was quality control. The goal was to identify and exclude from analysis array data with a sub-standard quality profile.

Step 2 was pre-processing and normalization. The goal was to create a normalized and scaled "analysis data set", amenable to inter-chip comparison. It comprised background noise estimation and subtraction, probe summarization and scaling.

Step 3 was exploration and description. The goal was to identify potential bias and sources of variability. It consisted of applying multivariate and univariate descriptive analysis techniques to identify influential covariates.

Step 4 was modeling and testing. The goal was to identify a list of candidate markers based on statistical evaluation of the difference in mean expression level between "Responders" (patients with "Partial Response" or "Complete Response" as best response) and "Non Responders" (patients "Progressive Disease" as best response). It consisted of fitting an adequate statistical model to each probe-set and deriving a measure of statistical significance.All analyses were performed using the R software package.

### Step 1 : Quality Control

The assessment of data quality was based on checking several parameters. These included standard Affymetrix GeneChip™ quality parameters, in particular: Scaling Factor, Percentage of Present Call, and Average Background. This step also included visual inspection of virtual chip images for detecting localized hybridization problems, and comparison of each chip to a virtual median chip for detecting any unusual departure from median behaviour. Inter-chip correlation analysis was also performed to detect outlier samples. In addition, ancillary measures of RNA quality obtained from analysis of RNA samples with the Agilent Bioanalyzer™ 2100 were taken into consideration.

Based on these parameters, data from 20 arrays were excluded from analysis. Thus data from a total of 102 arrays representing 102 patients was included in the analysis. The clinical description of these 102 patients set is reported in table 1.

**Table 1: Description of clinical characteristics of patients included in the analysis.**

| **Variable** | **Value** | **n=102** |
|---|---|---|
| | | n (%) |
| Best Response | N/A | 16 (15.7%) |
| | PD | 49 (48.0%) |
| | SD | 31 (30.4%) |
| | PR | 6 (5.9%) |
| | | |
| Clinical Benefit | NO | 81 (79.4%) |
| | YES | 21 (20.6%) |
| | | |
| SEX | FEMALE | 25 (24.5%) |
| | MALE | 77 (74.5%) |
| | | |
| ETHNICITY | CAUCASIAN | 65 (63.7%) |
| | ORIENTAL | 37 (36.3%) |
| | | |
| Histology | ADENOCARCINOMA | 35 (34.3%) |
| | SQUAMOUS | 53 (52.0%) |
| | OTHERS | 14 (13.7%) |
| | | |
| Ever-Smoking | NO | 20 (19.6%) |
| | YES | 82 (80.4%) |

### Step 2 : Data pre-processing and normalization

The rma algorithm (Irizarry, R.A., et al., *Summaries of Affymetrix GeneChip probe level data.* Nucl. Acids Res., 2003. **31**(4): p. e15) was used for pre-processing and normalization. The mas5 algorithm (AFFYMETRIX, *GeneChip*® *Expression: Data Analysis Fundamentals.* 2004, AFFYMETRIX) was used to make detection calls for the individual probe-sets. Probe-sets called "absent" or "marginal" in all samples were removed from further analysis; 5930 probe-sets were removed from analysis based on this criterion. The analysis data set therefore consisted of a matrix with 16353 (out of 22283) probe-sets measured in 102 patients.

### Step 3 : Data description and exploration

Descriptive exploratory analysis was performed to identify potential bias and major sources of variability. A set of covariates with a potential impact on gene expression profiles was screened. It comprised both technical and clinical variables. Technical covariates included: date of RNA processing (later referred to as batch), RIN (as a measure of RNA quality/integrity), Operator and Center of sample collection. Clinical covariates included: histology type, smoking status, tumour grade, performance score (Oken, M.M., et al., Toxicity and response criteria of the Eastern Cooperative Oncology Group. Am J Clin Oncol, 1982. 5(6): p. 649-55), demographic data, responder status and clinical benefit status.

The analysis tools included univariate ANOVA and principal component analysis. For each of these covariates, univariate ANOVA was applied independently to each probe-set.

A significant effect of the batch variable was identified. In practice, the batch variable captured differences between dates of sample processing and Affymetrix chip lot. After checking that the batch variable was nearly independent fromthe variables of interest, the batch effect was corrected using the method described in Johnson, W.E., C. Li, and A. Rabinovic, Adjusting batch effects in microarray expression data using empirical Bayes methods. Biostat, 2007. 8(1): p. 118-127.

The normalized data set after batch effect correction served as the analysis data set in subsequent analyses.

Histology and RIN were two additional important variables highlighted by the descriptive analysis.

### Step 4 : Data modeling and testing.

A linear model was fitted independently to each probe-set. Variables included in the model are reported in table 2. The model parameters were estimated by the maximum likelihood technique. The parameter corresponding to the "Response" variable (X1) was used to assess the difference in expression level between the group "responding" and "non responding" patients.

Table 2: Description of the variables included in the linear model. The linear model defined by these variables, including a normally distributed error term, was fitted to each probe-set.

| **Variable** | **Type** | **Values** |
|---|---|---|
| **gene expression** | **Dependent (Yᵢₚ)** | **Normalized log2 intensity of probe-set i in patient p.** |
| **Intercept** | **Overall mean (µ)** | |
| **Response** | **Predictor of interest (X1)** | **YES/NO** |
| **Histology** | **Adjustment Covariate (X2)** | **ADENOCARCINOMA/ SQUAMOUS / OTHERS** |
| **RACE** | **Adj. Cov. (X3)** | **ORIENTAL / CAUCASIAN** |
| **SEX** | **Adj Cov. (X4)** | **FEMALE / MALE** |
| **RIN** | **Adj Cov. (X5)** | **[2,...,7.9]** |

In this model, the response variable was defined as follows:
- Response = YES patients with partial response as their best response patients (n=6)
- Response= NO: patients with either Progressive disease (PD) as their best response or no tumour assessment (n=65)

Patients with a stable disease status were excluded (n=31). The rationale was that by focusing on patients with the more marked response to therapy, the non-responder group would become more homogeneous.

For each probe-set i, the aim of the statistical test was to reject the hypothesis that the mean expression levels in patients with response to treatment and patients without response to treatment are equal, taking into account the other adjustment covariates listed in table 2. Formally, the null hypothesis of equality was tested against a two sided alternative. Under the null hypothesis, the distribution of the Wald-statistic for this test follows a Chi-Square distribution with 64 degrees of freedom. The corresponding p-values are reported in table 3.

The choice of linear model was motivated by two reasons. Firstly, linear modeling is a versatile, well-characterized and robust approach that allows for adjustment of confounding variables when estimating the effect of the variable of interest. Secondly, given the sample size of 71, and the normalization and scaling of the data set, the normal distribution assumption was reasonable and justified.

The issue of multiple testing was dealt with by using a False Discovery Rate (FDR) criterion for identifying the list of differentially expressed genes (Benjamini et al., Journal of the Royal Statistical Society Series B-Methodological, 1995. 57(1): p. 289-300). Probe-sets with an FDR below the 0.3 threshold are declared significant. The 0.3 cut-off was chosen as a reasonable compromise between a rigorous correction for multiple testing with a stringent control of the risk of false positive and the risk of missing truly differential markers. The identified marker gene is reported in Table 3.

Table 3: Markers based on comparing "Responders" to "Progressors". Responders were defined as patients with Best Response equal to "Partial Response" (PR). "Progressors" were defined as patients having best response equal to "Progressive Disease"(PD) or no assessment available.

Patients with no tumour assessment were included in the "Progressors" groups because in the majority of cases, assessment was missing because of early withdrawal due to disease progression or death.

Column 1 is the Affymetrix identifier of the probe-set. Column 2 is the GenBank accession number of the corresponding gene sequence. Column 3 is the corresponding official gene name. Column 4 is the corresponding adjusted mean fold change in expression level between "responders" and "progressors", as estimated with the linear model. Column 5 is the p-value for the test of difference in expression level between "responders" and "progressors" as derived from the linear model. Column 6 is the 95% confidence interval for the adjusted mean fold change in expression level.

| Affymetrix Probe Set ID | GenBank | Gene | Adjusted Mean Fold Change | P-value | CI 95% |
|---|---|---|---|---|---|
| 204681_s_at | NM_012294 | RAPGEF5 | 2 | 2.10E-05 | 1.5, 2.7 |
| | XM_928602 | | | | |
| | XM_935655 | | | | |
| | XM_937844 | | | | |

For each probe-set, the assumption of homogeneity of variance was evaluated using Fligner-Killeen tests based on the model residuals. The analysis consisted of three steps:
Test all categorical variables for equality of residual variance between their levels
Note the variable V with the least p-value
If the least p-value is less than 0.001, re-fit the model allowing the different level of variables V to have a different variance.

### Further statistical anal ysis

For the selected candidate marker RAPGEF5, the following additional analyses were performed in a validated environment by an independent statisticians :
- Univariate Cox Regression for PFS (Progression free survival) from Primary Affymetrix Analysis,
- Univariate Logistic Regression for Response from Primary Affymetrix Analysis, and
- Univariate Cox Regression for Survival from Primary Affymetrix Analysis

The results of these analysis are presented below. They are consistent with the results of the primary analysis and confirm the choice of the selected marker.

Results: Univariate Cox Regression for PFS (Progression free survival) from Primary Affymetrix Analysis:

| **Gene** | **No. of patients** | **Hazard ratio** | **95 % CI for Hazard ratio** | **p-Value** |
|---|---|---|---|---|
| PSPH | 102 | 0.44 | 0.28; 0.69 | 0.0003 |

Results: Univariate Cox Regression for Response from Primary Affymetrix Analysis:

| **Gene** | **No. of patients** | **Odds ratio** | **95 % CI for Odds ratio** | **p-Value** |
|---|---|---|---|---|
| PSPH | 102 | 95.77 | 5.84; >1000 | 0.0014 |

Results: Univariate Cox Regression for Survival from Primary Affymetrix Analysis:

| **Gene** | **No. of patients** | **Hazard ratio** | **95 % CI for Hazard ratio** | **p-Value** |
|---|---|---|---|---|
| PSPH | 102 | 0.47 | 0.28; 0.78 | 0.0040 |

### qRT-PCR

cDNA was synthesized using SuperScript^{™} III First-strand Synthesis SuperMix for qRT-PCR (Invitrogen, CA, USA) according to the manufacturer's instructions but without inclusion of an RNase H digest.

Quantitative PCR was performed using TaqMan^{®} Gene Expression Assays on an ABI PRISM^{®} 7900HT Sequence Detection System according to the manufacturer's recommendations (Applied Biosystems, CA, USA). All assays were performed in triplicate.

The Gene Expression Assays used Hs00920287_ml [*RAPGEF5*]) and were chosen so that the primers and probes crossed exon boundaries or were within the Affymetrix Genechip^{®} probe sequence of interest. Two house-keeping genes were included as endogenous controls: beta-2-microglobulin (*B2M;* Assay Hs99999907_ml) and hypoxanthinephosphoribosyl transferase (*HPRT;* Assay Hs99999909_ml).

All runs included a calibrator sample (MVP^{™} total RNA from human adult lung; Stratagene, CA, USA) and a standard curve. Universal Human Reference total RNA (Stratagene, CA, USA) was used as template for *EGFR* and *PSPH* standard curves. *RAPGEF5* mRNA was present at an unacceptably low level in Universal Human Reference total RNA, and so MVP^{™} total RNA from human adult lung was used to construct standard curves for this gene. All samples were measured in triplicate.Relative quantification was performed using the -ΔCt method.

### Results

As reported previously, Affymetrix Genechip^{®} gene expression profiles were determined for 102 patients included in this study. Among these patients, qRT-PCR results were obtained for 75 (table 4). The demographics and clinical characteristics of the patients with qRT-PCR results were similar to those of the entire population (n=264) and of the patients with Genechip^{®} gene expression profiles available.

**Table 4: Baseline characteristics: patients with qRT-PCR analyses (n=75)**

| **Characteristic** | |
|---|---|
| Age (median, range) | 62 (39-85) |
| Gender; n (%) | |
| Male | 19 (25) |
| Female | 56 (75) |
| ECOG performance status; n (%) | |
| 0 | 7 (9) |
| 1 | 45 (60) |
| 2 | 23 (31) |
| Histology; n (%) | |
| Adenocarcinoma | 27 (36) |
| Squamous-cell carcinoma | 34 (45) |
| Large-cell carcinoma | 2 (3) |
| Other | 12 (16) |
| Disease stage; n (%) | |
| IIIB | 22 (29) |
| IV | 53 (71) |
| Number of prior chemotherapy regimens; n (%) | |
| 0 | 19 (25) |
| 1 | 36 (48) |
| ≥2 | 20 (27) |
| Ethnicity; n (%) | |
| Caucasian | 51 (68) |
| Asian | 24 (32) |
| Smoking history; n (%) | |
| Never | 12 (16) |
| Current | 24 (32) |
| Former | 39 (52) |

Of the 75 patients with qRT-PCR results, 4 (5%) had partial response (PR), 23 (31%) had SD, 39 (52%) had PD, and 9 (12%) were not evaluable. These results were very similar to those observed in the entire study population (n=264).

### Correlation between Affymetrix Genechip^{®} data, qRT-PCR data and clinical outcomes

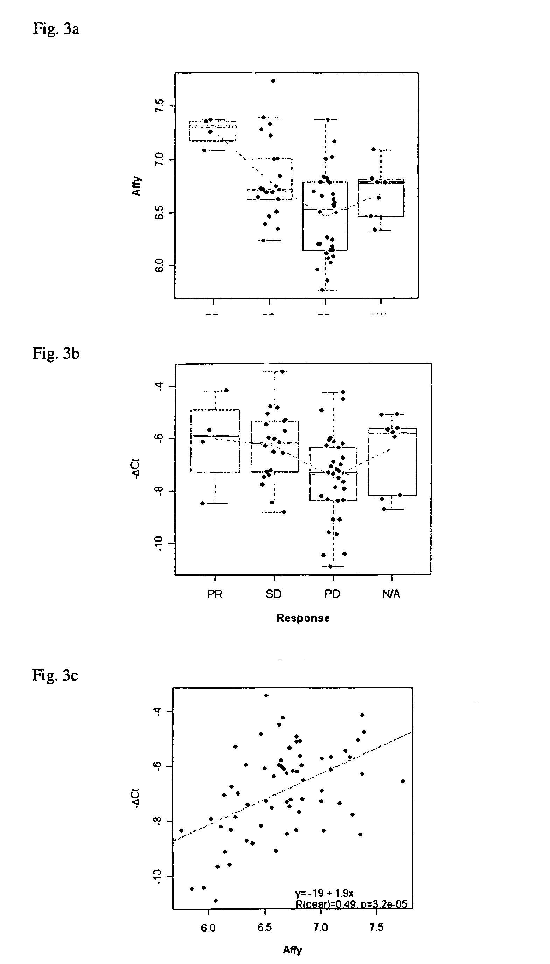

Figure 3 shows relative mRNA levels for *RAPGEF5* in individual patients, as assessed by Affymetrix GeneChip^{®} profiling and qRT-PCR. Fig. 3a shows expression levels versus clinical outcome for Genechip^{®} profiling and Fig. 3b shows expression levels versus clinical outcome for qRT-PCR.

Figure 3c shows the correlation between Genechip^{®} and qRT-PCR measurements for *RAPGEF5.*

There was a correlation between Genechip^{®} and qRT-PCR measurements for RAPGEF5. The association between clinical outcome with erlotinib and RAPGEF5 mRNA levels assessed by qRT-PCR was somewhat poorer than that observed with Genechip^{®} profiling (Figure 3b).

### Identification of genes associated with response to erlotinib

Responders were defined as patients whose best response was partial response, while "progressors" were defined as patients having either progressive disease or for whom no assessment was made (in most cases as a result of early withdrawal due to disease progression or death). Thus in this model 6 "responders" were compared to 65 "progressors".

A linear model was fitted independently to each of the 16353 remaining probe-sets used in the analysis after removal of those probe-sets that were not present in any sample from the total 22283 on the HG-U133A microarray. A p-value was calculated for the difference in expression between response and non-response for each probe-set. A false discovery rate (FDR) of 0.3 was applied to correct for multiple testing. The marker identified from this analysis is shown in Table 3.

### Discussion

Targeting the Epidermal Growth Factor Receptor (EGFR) as a means of cancer therapy was proposed based on its ubiquitous aberrant expression in several epithelial cancers (Davies & Chamberlin 1996, Mendelsohn, 2002). EGFR is implicated in the pathogenesis and progression of many tumours including 40-80% of NSCLC tumours (Baselga & Albanell, 2002; Normanno et al, 2006), as a result of activating mutations in the tyrosine kinase domain and / or its amplification. Upon activation, the receptor undergoes dimerization, resulting in phosphorylation of downstream targets with roles in cellular proliferation, metastasis, inhibition of apoptosis and neoangiogenesis (Mendelsohn & Baselga, 2003).

Two major classes of EGFR inhibitors have been developed, monoclonal antibodies targeting the extracellular domain of the receptor, and small molecule tyrosine kinase inhibitors targeting the catalytic domain of the receptor. The latter include erlotinib which competes with ATP for the intracellular binding site.

It has emerged in recent years that several factors play a role in sensitivity to erlotinib including female gender, non-smoker status, Asian origin and adenocarcinoma histology; given that enhanced response rates are evident in such clinical subsets of patients, extensive efforts are ongoing to elucidate predictive molecular markers for patient stratification. Mutations in the *EGFR,* amplification of the *EGFR* gene locus and overexpression of EGFR on the protein level, have all been associated with response to varying degrees, though these are not the only molecular determinants of response.

By analyzing tissue samples with high-density oligonucleotide microarray technology, and applying statistical modeling to the data, we have been able to identify a gene whose expression level is predictive of response to erlotinib (comparison of PR versus PD) (Table 3). This gene is RAPGEF5 which is located at 7p15.3 is also overexpressed in responders (2.0 fold upregulated; p=0.000021).

RAPGEF5 was found to be approximately 2-fold upregulated in PR as compared to PD only (p=0.000021) in our analysis. RAPGEF5 is a guanine nucleotide exchange factor (GEF) for Rap1, a member of the RAS subfamily of GTPases that function in signal transduction as. GEFs provide the pivotal link between cell surface receptors and GTPase activation by cycling between inactive GDP- and active GTP-bound states. Rap1 is closely related to Ras and implicated in the regulation of a variety of cellular processes including the control of platelet activation, T-cell anergy, B-cell activation, neuronal differentiation and is involved in the control of cell adhesion and the regulation of integrin activation. Some of these processes are also likely to be of relevance for the progression of NSCLC. RAPGEF5 may have an indirect role in angiogenesis through its control of Rap1, which has recently been shown to be a pivotal regulator of vascular endothelial cell chemotaxis. Promotion of neo-angiogenesis is a prominent feature of neoplastic growth; bevacizumab, an agent that specifically targets one of the key players in angiogenesis, the vascular endothelial growth factor (VEGF) has recently been shown to extend survival in combination with chemotherapy in non-squamous NSCLC. The precise role of RAPGEF5 in carcinogenesis remains to be determined, but it may provide an additional link into the MAPK pathway through which EGFR is known to signal.

**Table 5: List of RAPGEF5 variants and their corresponding Sequence identification number**

| **GenBank Accession number** | **Gene** | **Sequence identification number** |
|---|---|---|
| NM_012294 | RAPGEF5 | Seq. Id. No. 1 |
| XM_928602 | RAPGEF5_variant_1 | Seq. Id. No. 2 |
| XM_935655 | RAPGEF5_variant_6 | Seq. Id. No. 3 |
| XM_937844 | RAPGEF5_variant_11 | Seq. Id. No. 4 |

## Claims

1. An *in vitro* method of predicting the response of a NSCLC patient to treatment with erlotinib comprising: determining a mRNA expression level of a RAPGEF5 gene in a tumour sample of a patient and comparing the mRNA expression level of the RAPGEF5 gene to a value representative of a mRNA expression level of the RAPGEF5 gene in tumours of a non responding patient population, wherein a higher mRNA expression level of the RAPGEF5 gene in the tumour sample of the patient is indicative for a patient who will respond to the treatment.

2. The method of claim 1, wherein the mRNA expression level is determined by microarray technology or any technology that measures RNA expression.

3. The method of claims 1 to 2, wherein the RAPGEF5 gene shows a 1.5 to 2.7 or more fold higher mRNA expression in the tumour sample of the responding patient compared to to the value representative of a mRNA expression level of the RAPGEF5 gene in tumours of a non responding patient population.

4. Use of a RAPGEF5 gene for predicting the response of a NSCLC patient to erlotinib treatment.

5. Erlotinib for use in treating a NSCLC patient identified by a method of claims 1 to 3 as a patient who will respond to the treatment comprising administering erlotinib to the patient.

## Patentansprüche

1. *In-vitro*-Verfahren zur Vorhersage, ob ein NSCLC-Patient auf Erlotinib-Behandlung anspricht, umfassend: Bestimmen des mRNA-Expressionsspiegels eines RAPGEF5-Gens in einer Tumorprobe des Patienten und Vergleichen des mRNA-Expressionsspiegels des RAPGEF5-Gens mit einem Wert, der repräsentativ ist für einen mRNA-Expressionsspiegel des RAPGEF5-Gens in Tumoren von einer Patientenpopulation, die nicht auf die Behandlung anspricht, wobei ein erhöhter mRNA-Expressionsspiegel des RAPGEF5-Gens in der Tumorprobe des Patienten ein Anzeichen dafür ist, dass der Patient auf die Behandlung anspricht.

2. Verfahren nach Anspruch 1, wobei der mRNA-Expressionsspiegel durch Microarray-Technologie bestimmt wird, oder eine Technologie, die die RNA-Expression misst.

3. Verfahren nach Anspruch 1 oder 2, wobei das RAPGE1F5-Gen eine 1,5-fache bis 2,7-fache oder höhere mRNA-Expression in der Tumorprobe des Patienten, der auf eine Behandlung anspricht, zeigt, verglichen mit einem Wert, der repräsentativ ist für einen mRNA-Expressionsspiegel des RAPGEF5-Gens in Tumoren von einer Patientenpopulation, die nicht auf die Behandlung anspricht.

4. Verwendung eines RAPGEF5-Gens zur Vorhersage, ob ein NSCLC-Patient auf Erlotinib-Behandlung anspricht.

5. Erlotinib zur Verwendung in der Behandlung eines NSCLC-Patienten, der durch ein Verfahren nach einem der Ansprüche 1 bis 3 als ein Patient identifiziert ist, der auf die Behandlung anspricht, umfassend die Verabreichung von Erlotinib an den Patienten.

## Revendications

1. Procédé *in vitro* de prédiction de la réponse d'un patient souffrant de NSCLC au traitement avec l'erlotinib, comprenant: la détermination du niveau d'expression de l'ARNm d'un gène de RAPGEF5 dans un échantillon de tumeur du patient et la comparaison du niveau d'expression de l'ARNm du gène de RAPGEF5 à une valeur représentative du niveau d'expression de l'ARNm du gène de RAPGEF5 dans les tumeurs d'une population de patients non répondeurs, un niveau d'expression de l'ARNm d'un gène de RAPGEF5 plus élevé dans l'échantillon de tumeur du patient indiquant un patient qui répondra au traitement.

2. Procédé selon la revendication 1, dans lequel le niveau d'expression de l'ARNm est déterminé par une technologie de micropuce ou par toute technologie qui mesure l'expression d'un ARN.

3. Procédé selon les revendications 1 à 2, dans lequel le gène de RAPGEF5 présente une expression d'ARNm 1,5 à au moins 2,7 fois supérieure dans l'échantillon de tumeur du patient répondeur par comparaison à la valeur représentative du niveau d'expression de l'ARNm du gène de RAPGEF5 dans les tumeurs d'une population de patients non répondeurs.

4. Utilisation d'un gène de RAPGEF5 pour la prédiction de la réponse d'un patient souffrant de NSCLC au traitement avec l'erlotinib.

5. Erlotinib à utiliser dans le traitement d'un patient souffrant de NSCLC identifié par un procédé des revendications 1 à 3 en tant que patient qui répondra au traitement, comprenant l'administration d'erlotinib au patient.
